# EUROPEAN PATENT SPECIFICATION

(11) **EP 3 552 554 B1**
(45) Date of publication and mention of the grant of the patent: **27.03.2024**
(21) Application number: 17877407.1
(22) Date of filing: 20.11.2017
(51) Int. Cl.: A61B 8/14, A61B 8/08, A61B 8/00

(54) **ULTRASONIC DIAGNOSIS APPARATUS AND METHOD FOR CONTROLLING ULTRASONIC DIAGNOSIS APPARATUS**
ULTRASCHALLDIAGNOSEVORRICHTUNG UND VERFAHREN ZUR STEUERUNG DER ULTRASCHALLDIAGNOSEVORRICHTUNG
APPAREIL DE DIAGNOSTIC À ULTRASONS ET PROCÉDÉ DE COMMANDE D'UN APPAREIL DE DIAGNOSTIC À ULTRASONS

(30) Priority: 06.12.2016 JP 2016236580
(43) Date of publication of application: 16.10.2019
(60) Divisional application: 24154651.4
(73) Proprietor: FUJIFILM Corporation, Tokyo 106-8620 (JP)
(72) Inventor: MATSUMOTO Tsuyoshi, Ashigara-kami-gun Kanagawa 258-8538 (JP); INOUE Tomoki, Ashigara-kami-gun Kanagawa 258-8538 (JP); YAMAMOTO Hiroaki, Ashigara-kami-gun Kanagawa 258-8538 (JP)
(74) Representative: Klunker IP Patentanwälte PartG mbB
(86) International application number: PCT/JP2017/041640
(87) International publication number: WO 2018/105366

(56) References cited:
- WO-A1-2014/097090
- JP-A- 2005 124 712
- JP-B2- 5 842 810
- US-A1- 2004 019 270
- US-A1- 2016 007 955

## Description

### BACKGROUND OF THE INVENTION

### 1. Field of the Invention

The present invention relates to an ultrasound diagnostic apparatus and a control method of an ultrasound diagnostic apparatus and in particular, to an ultrasound diagnostic apparatus for notifying an operator of the posture angle of an ultrasound probe with respect to an examination part of a subject and a control method of the ultrasound diagnostic apparatus.

### 2. Description of the Related Art

Conventionally, an ultrasound diagnostic apparatus is known as an apparatus for acquiring an image of the inside of a subject. In general, an ultrasound diagnostic apparatus comprises an ultrasound probe comprising a transducer array in which a plurality of elements are arranged. In a state in which the ultrasound probe is in contact with the body surface of the subject, an ultrasound beam is transmitted from the transducer array to the inside of the subject and ultrasound echoes from the subject are received in the transducer array, so that element data is acquired. In addition, the ultrasound diagnostic apparatus electrically processes the acquired element data to generate an ultrasound image of the relevant part of the subject.

It is known that the clarity of an imaging part in the ultrasound image captured by such an ultrasound diagnostic apparatus changes according to the posture angle of the ultrasound probe in contact with the body surface of the subject. For this reason, at the time of examining the part of the subject using the ultrasound diagnostic apparatus, in order to clearly image the examination part of the subject, the operator needs to bring the ultrasound probe into contact with the body surface of the subject and tilt the ultrasound probe to find the optimal posture angle of the ultrasound probe.

Therefore, various ultrasound diagnostic apparatuses capable of acquiring the appropriate posture angle of the ultrasound probe and clearly imaging the examination part of the subject have been proposed. For example, an ultrasound diagnostic apparatus disclosed in JP5842810B displays a reference probe icon, which shows an angle of an ultrasound probe in a case where an ultrasound image was captured in the past or a posture angle set in advance according to the current diagnostic mode, and a current probe icon, which shows a posture angle of an ultrasound probe being moved by the operator, simultaneously with the ultrasound image. By moving the ultrasound probe so that the reference probe icon and the current probe icon match each other, the operator can fix the ultrasound probe at the posture angle set in advance according to the current diagnostic mode and image the examination part of the subject. US 2004/019270 is directed to an ultrasonic diagnosis apparatus, an ultrasonic probe, and an ultrasonic imaging assisting method that make manipulations easier and adequate for non-specialized or less experienced physicians or technicians using a navigation information generating unit that generates navigation information used to assist a manipulation of acquiring an ultrasonic image identical or similar to a reference image.

### SUMMARY OF THE INVENTION

Incidentally, since the structure and the position of an examination part vary depending on a subject, an appropriate posture angle of an ultrasound probe differs depending on a subject. For this reason, even in a case where the posture angle of the ultrasound probe is determined with reference to a reference posture angle set in advance using the technique disclosed in JP5842810B, it is not possible to clearly image the examination part of the subject.

For example, in the extended Focused Assessment with Sonography for Trauma (eFAST) examination for continuously examining a plurality of examination parts for initial examination of an injured patient in emergency, it is requested to quickly perform ultrasound examination on the plurality of examination parts. In the case of such an examination, the examination has been performed on a subject having no past ultrasound diagnostic data in many cases, and the examination time is short. Therefore, even in a case where the technique disclosed in JP5842810B is used, it is difficult to clearly image the examination part of the subject.

The present invention has been made in order to solve such a conventional problem, and it is an object of the present invention to provide an ultrasound diagnostic apparatus and a control method of an ultrasound diagnostic apparatus capable of clearly imaging an examination part even in a case where a subject is changed.

In order to achieve the aforementioned object, an ultrasound diagnostic apparatus of the present invention comprises: an ultrasound probe; an image generation unit that generates an ultrasound image by transmitting an ultrasound beam from the ultrasound probe toward a subject and receiving an ultrasound beam reflected from the subject; a probe posture angle detection unit that detects a posture angle of the ultrasound probe; an image analysis unit that calculates a likelihood of an imaging part in the ultrasound image with respect to an examination part by analyzing the ultrasound image generated by the image generation unit; a recommended posture angle generation unit that sets, as a recommended posture angle, the posture angle detected by the probe posture angle detection unit in a case where the likelihood calculated by the image analysis unit is maximized while the posture angle detected by the probe posture angle detection unit is being changed over a predetermined angle range including a basic posture angle of the ultrasound probe, which is set corresponding to the examination part, by bringing the ultrasound probe into contact with a body surface of the subject and tilting the ultrasound probe by an operator; and a recommended posture angle notification unit that notifies the operator of the recommended posture angle generated by the recommended posture angle generation unit.

It is preferable that the recommended posture angle generation unit sets, as the recommended posture angle, the posture angle detected by the probe posture angle detection unit in a case where the likelihood calculated by the image analysis unit is maximized while the posture angle detected by the probe posture angle detection unit is changing once over the angle range.

Alternatively, it is preferable that the recommended posture angle generation unit sets, as the recommended posture angle, the posture angle detected by the probe posture angle detection unit in a case where the likelihood calculated by the image analysis unit is maximized while the posture angle detected by the probe posture angle detection unit is changing in a reciprocating manner over the angle range.

It is preferable to further comprise: a probe angular speed detection unit that detects an angular speed of the ultrasound probe; and a probe tilt warning unit that warns the operator that the ultrasound probe is to be continuously tilted in a case where the posture angle detected by the probe posture angle detection unit is within the angle range and the angular speed detected by the probe angular speed detection unit becomes equal to or less than a predetermined value.

It is preferable to further comprise: a display unit that displays the ultrasound image generated by the image generation unit; and a display control unit that controls display on the display unit.

It is preferable that the display control unit displays, on the display unit, the likelihood calculated by the image analysis unit, the posture angle detected by the probe posture angle detection unit, the basic posture angle, and the recommended posture angle generated by the recommended posture angle generation unit.

It is preferable that the display control unit displays the posture angle detected by the probe posture angle detection unit, the basic posture angle, and the recommended posture angle generated by the recommended posture angle generation unit, on the display unit, as numerical values or graph images.

A control method of an ultrasound diagnostic apparatus of the present invention comprises: generating an ultrasound image by transmitting an ultrasound beam from an ultrasound probe toward a subject and receiving an ultrasound beam reflected from the subject; detecting a posture angle of the ultrasound probe; calculating a likelihood of an imaging part in the ultrasound image with respect to an examination part by analyzing the generated ultrasound image; setting, as a recommended posture angle, the posture angle in a case where the calculated likelihood is maximized while the detected posture angle is being changed over a predetermined angle range including a basic posture angle of the ultrasound probe, which is set corresponding to the examination part, by bringing the ultrasound probe into contact with a body surface of the subject and tilting the ultrasound probe by an operator; and notifying the operator of the recommended posture angle.

According to the present invention, the ultrasound diagnostic apparatus has the recommended posture angle generation unit that sets, as a recommended posture angle, the posture angle of the ultrasound probe in a case where the likelihood of the imaging part in the ultrasound image is maximized, and the operator is notified of the recommended posture angle. Therefore, even in a case where the subject is changed, it is possible to image the examination part clearly.

### BRIEF DESCRIPTION OF THE DRAWINGS

Fig. 1 is a block diagram showing the configuration of an ultrasound diagnostic apparatus according to a first embodiment of the present invention.
Fig. 2 is a block diagram showing the internal configuration of a reception circuit shown in Fig. 1.
Fig. 3 is a block diagram showing the internal configuration of an image generation unit shown in Fig. 1.
Fig. 4 is a flowchart showing the operation of the ultrasound diagnostic apparatus according to the first embodiment of the present invention.
Fig. 5 is a conceptual diagram showing an example of a screen display during ultrasound diagnosis on a display unit shown in Fig. 1.
Fig. 6 is a conceptual diagram showing an example of a display of a radar chart on the display unit shown in Fig. 1.
Fig. 7 is a flowchart showing the operation of an ultrasound diagnostic apparatus according to a second embodiment of the present invention.
Fig. 8 is a block diagram showing the configuration of an ultrasound diagnostic apparatus according to a third embodiment of the present invention.
Fig. 9 is a flowchart showing the operation of the ultrasound diagnostic apparatus according to the third embodiment of the present invention.
Fig. 10 is a flowchart showing the operation of an ultrasound diagnostic apparatus according to a fourth embodiment of the present invention.

### DESCRIPTION OF THE PREFERRED EMBODIMENTS

Hereinafter, embodiments of the present invention will be described with reference to the accompanying diagrams.

### First embodiment

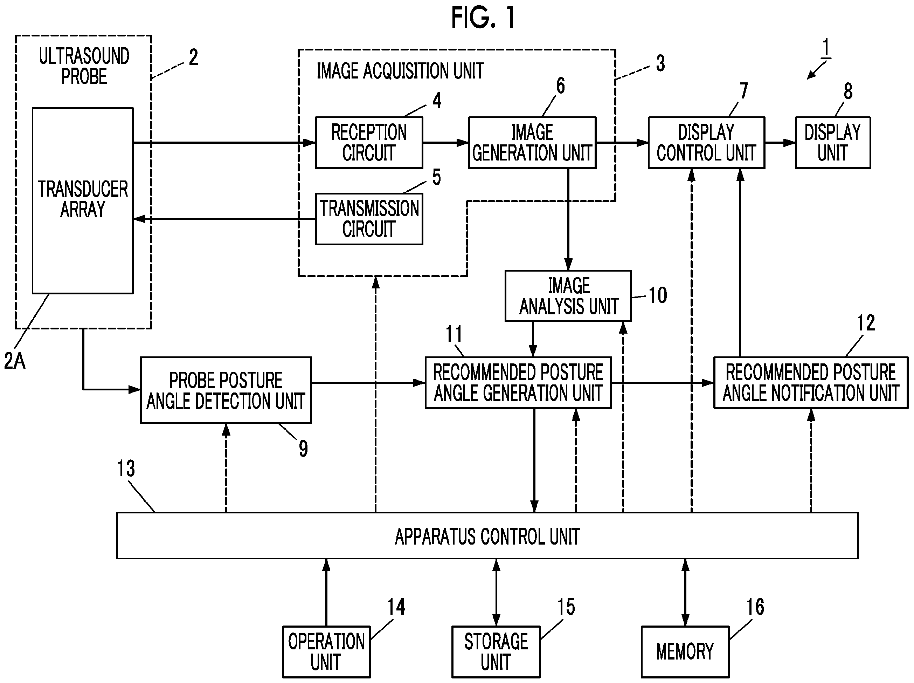

Fig. 1 shows the configuration of an ultrasound diagnostic apparatus according to a first embodiment of the present invention. An ultrasound diagnostic apparatus 1 comprises an ultrasound probe 2 in which a transducer array 2A is built, and a display control unit 7 and a display unit 8 are sequentially connected to the ultrasound probe 2 through an image acquisition unit 3.

The image acquisition unit 3 has a reception circuit 4 and a transmission circuit 5, which are connected to the transducer array 2A of the ultrasound probe 2, and an image generation unit 6 connected to the reception circuit 4, and the display control unit 7 is connected to the image generation unit 6. The ultrasound probe 2 comprises a probe posture angle detection unit 9, and a recommended posture angle generation unit 11 is connected to the probe posture angle detection unit 9. An image analysis unit 10 is connected to the image generation unit 6 of the image acquisition unit 3, and the recommended posture angle generation unit 11 is connected to the image analysis unit 10. A recommended posture angle notification unit 12 is connected to the recommended posture angle generation unit 11, and the recommended posture angle notification unit 12 is connected to the display control unit 7.

An apparatus control unit 13 is connected to the image acquisition unit 3, the display control unit 7, the probe posture angle detection unit 9, the image analysis unit 10, the recommended posture angle generation unit 11, and the recommended posture angle notification unit 12, and an operation unit 14, a storage unit 15, and a memory 16 are connected to the apparatus control unit 13. The apparatus control unit 13 and the storage unit 15 are connected to each other so that information can be transmitted and received bidirectionally, and the apparatus control unit 13 and the memory 16 are connected to each other so that information can be transmitted and received bidirectionally.

The transducer array 2A of the ultrasound probe 2 shown in Fig. 1 has a plurality of elements (ultrasound transducer) arranged in a one-dimensional or two-dimensional manner. According to a driving signal supplied from the transmission circuit 5, each of the elements transmits an ultrasound wave and receives an ultrasound echo from the subject and outputs a reception signal. For example, each element is formed by using a transducer in which electrodes are formed at both ends of a piezoelectric body formed of piezoelectric ceramic represented by lead zirconate titanate (PZT), a polymer piezoelectric element represented by poly vinylidene di fluoride (PVDF), piezoelectric single crystal represented by lead magnesium niobate-lead titanate (PMN-PT), or the like.

In a case where a pulsed or continuous-wave voltage is applied to the electrodes of such a transducer, the piezoelectric body expands and contracts to generate pulsed or continuous-wave ultrasound waves from each transducer. From the combined wave of these ultrasound waves, an ultrasound beam is formed. The respective transducers expand and contract by receiving the propagating ultrasound waves, thereby generating electric signals. These electric signals are output, as reception signals of the ultrasound waves, from each transducer to the reception circuit 4.

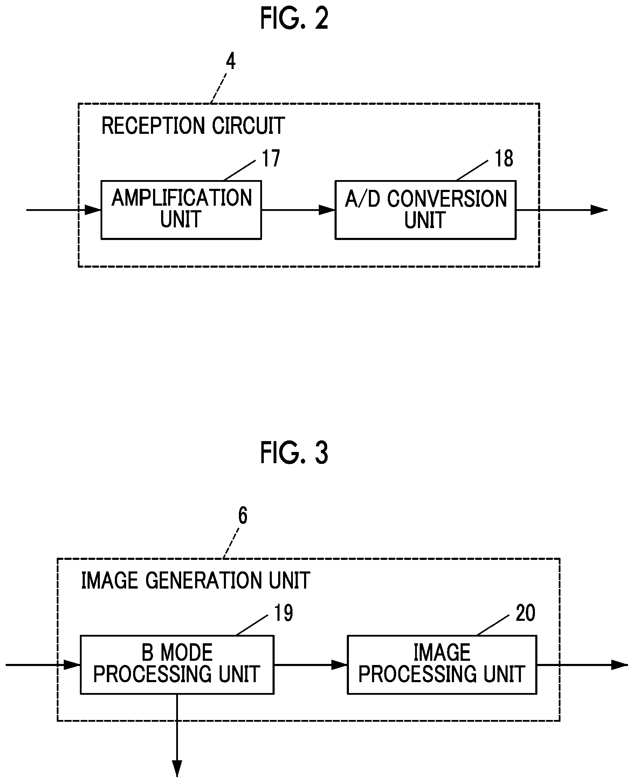

As shown in Fig. 2, the reception circuit 4 of the image acquisition unit 3 has a configuration in which an amplification unit 17 and an analog/digital (A/D) conversion unit 18 are connected in series to each other. In the reception circuit 4, the amplification unit 17 amplifies the reception signal output from each element of the transducer array 2A, and the A/D conversion unit 18 converts the amplified signal into a digital signal to obtain element data and outputs the obtained element data to the image generation unit 6.

The transmission circuit 5 of the image acquisition unit 3 includes, for example, a plurality of pulse generators. Based on a transmission delay pattern selected according to the control signal from the apparatus control unit 13, the transmission circuit 5 adjusts the amount of delay of each driving signal so that ultrasound waves transmitted from the plurality of elements of the transducer array 2A form an ultrasound beam, and supplies the obtained signals to the plurality of elements.

As shown in Fig. 3, the image generation unit 6 of the image acquisition unit 3 has a configuration in which a brightness mode (B mode) processing unit 19 and an image processing unit 20 are sequentially connected in series to each other.

Based on the reception delay pattern selected according to the control signal from the apparatus control unit 13, the B mode processing unit 19 performs reception focusing processing in which delays are given to respective pieces of element data according to the set sound speed and addition (phasing addition) is performed. Through the reception focusing processing, a sound ray signal with narrowed focus of the ultrasound echo is generated. The B mode processing unit 19 generates a B mode image signal, which is tomographic image information regarding tissues inside the subject, by correcting the attenuation of the sound ray signal due to the propagation distance according to the depth of the reflection position of the ultrasound wave and then performing envelope detection processing. The B mode image signal generated by the B mode processing unit 19 is output to the display control unit 7 and the image analysis unit 10.

The image processing unit 20 converts (raster conversion) the B mode image signal generated by the B mode processing unit 19 into an image signal according to the normal television signal scanning method and performs various kinds of required image processing, such as gradation processing, on the B mode image signal, and then outputs the B mode image signal to the display control unit 7.

As shown in Fig. 1, the display control unit 7 of ultrasound diagnostic apparatus 1 displays an ultrasound diagnostic image on the display unit 8 based on the B mode image signal acquired by the image acquisition unit 3.

The display unit 8 includes, for example, a display device, such as a liquid crystal display (LCD), and displays an ultrasound diagnostic image under the control of the apparatus control unit 13.

The ultrasound probe 2 comprises the probe posture angle detection unit 9, and the probe posture angle detection unit 9 detects the posture angle of the ultrasound probe 2 that is brought into contact with the body surface of the subject and tilted by the operator during ultrasound diagnosis. In this case, the probe posture angle detection unit 9 detects the angles of three components defined based on three axes perpendicular to each other in a three-dimensional space. Here, the three axes perpendicular to each other in the three-dimensional space are defined as follows. That is, a direction perpendicular to the ultrasound wave emission surface of the ultrasound probe 2 is defined as the x axis, and directions perpendicular to each other along the ultrasound wave emission surface of the ultrasound probe 2 are defined as the y axis and the z axis, respectively. In this case, the probe posture angle detection unit 9 detects an angle in a case where the x axis is a horizontal direction as a pitch angle, detects an angle in a case where the y axis is a horizontal direction as a roll angle, and detects an angle in a case where the z axis is a vertical direction as a yaw angle.

The probe posture angle detection unit 9 is not particularly limited as long as the posture angle of the ultrasound probe 2 can be detected. Although not shown, the following description will be given on the assumption that the probe posture angle detection unit 9 is attached to the ultrasound probe 2, includes three angle sensors that detect a pitch angle, a roll angle, and a yaw angle as electric signals, and detects the posture angle of the ultrasound probe 2 as angle information by converting electric signals acquired from the angle sensors into angle information.

The image analysis unit 10 performs image analysis, such as pattern recognition, on the B mode image signal generated by the B mode processing unit 19 of the image generation unit 6 of the image acquisition unit 3, thereby calculating the likelihood of an imaging part in the ultrasound image with respect to the examination part. Here, the likelihood of the imaging part in the ultrasound image with respect to the examination part in the present invention is a numerical value indicating the likelihood of the imaging part in the ultrasound image with respect to the examination part, and it can be determined that, as the numerical value of the likelihood becomes larger, the probability that the imaging part in the ultrasound image is the examination part becomes higher, that is, the imaging part in the ultrasound image is clearly imaged as the examination part. For example, in a case where an ultrasound image of the heart is captured using the ultrasound diagnostic apparatus 1, it can be determined that, as the numerical value of the likelihood of an imaging part in the ultrasound image with respect to the heart that is an examination part becomes larger, the imaging part in the ultrasound image is more clearly imaged as the heart that is an examination part.

The recommended posture angle generation unit 11 is a characteristic element of the present invention. The recommended posture angle generation unit 11 sets, as a recommended posture angle, a posture angle in a case where the likelihood of the imaging part in the ultrasound image calculated by the image analysis unit 10 is maximized while the posture angle of the ultrasound probe 2 detected by the probe posture angle detection unit 9 is changing over a predetermined angle range including the basic posture angle of the ultrasound probe 2. Here, the angle range refers to an angle range having two different angles as upper and lower limits. The basic posture angle is a posture angle of the ultrasound probe 2 set in advance corresponding to the examination part, and is a posture angle of the ultrasound probe 2 at which the examination part of the subject can be imaged so that the imaging part in the ultrasound image becomes clear with respect to the general structure and position of the examination part. Compared with the basic posture angle, the recommended posture angle is generated based on the posture angle of the ultrasound probe 2 during ultrasound diagnosis and the image analysis result of the captured ultrasound image. Therefore, the recommended posture angle is a posture angle of the ultrasound probe 2 at which the examination part of the subject can be imaged so that the imaging part in the ultrasound image becomes clear with respect to the unique structure and position of the examination part of the subject who is a target of ultrasound diagnosis.

The recommended posture angle notification unit 12 notifies the operator of the recommended posture angle generated by the recommended posture angle generation unit 11. The notification method of the recommended posture angle notification unit 12 is not particularly limited as long as the operator can be notified of the recommended posture angle. Hereinafter, the following description will be given on the assumption that the recommended posture angle notification unit 12 transmits instruction information for displaying the recommended posture angle on the display unit 8 to the display control unit 7.

The apparatus control unit 13 controls each unit of the ultrasound diagnostic apparatus 1 based on a command input by the operator through the operation unit 14.

The operation unit 14 is for the operator to perform an input operation, and can be configured to comprise a keyboard, a mouse, a trackball, a touch panel, and the like.

The storage unit 15 stores an operation program and the like of the ultrasound diagnostic apparatus 1, and recording media, such as a hard disc drive (HDD), a solid state drive (SSD), a flexible disc (FD), a magneto-optical disc (MO), a magnetic tape (MT), a random access memory (RAM), a compact disc (CD), a digital versatile disc (DVD), a secure digital card (SD card), and a universal serial bus memory (USB memory), or a server can be used.

The memory 16 stores an examination part label for designating an examination part, the basic posture angle of the ultrasound probe 2, the recommended posture angle of the ultrasound probe 2, the posture angle of the ultrasound probe 2 detected by the probe posture angle detection unit 9, the likelihood of an imaging part calculated by the image analysis unit 10, and the like. As the memory 16, similarly to the storage unit 15, recording media, such as an HDD, an SSD, an FD, an MO disk, an MT, a RAM, a CD, a DVD, an SD card, and a USB memory, a server, and the like can be used.

The image generation unit 6 of the image acquisition unit 3, the display control unit 7, the image analysis unit 10, the recommended posture angle generation unit 11, the recommended posture angle notification unit 12, and the apparatus control unit 13 are configured by a central processing unit (CPU) and an operation program causing the CPU to execute various kinds of processing. However, these may also be configured using a digital circuit and a computer. The image generation unit 6, the display control unit 7, the image analysis unit 10, the recommended posture angle generation unit 11, and the apparatus control unit 13 can also be integrated partially or entirely in one CPU. Although the probe posture angle detection unit 9 may be configured to include a CPU and an operation program causing the CPU to execute various kinds of processing, these may also be configured using a digital circuit and a computer, and can be partially integrated in one CPU together with the image generation unit 6, the display control unit 7, the image analysis unit 10, the recommended posture angle generation unit 11, and the apparatus control unit 13.

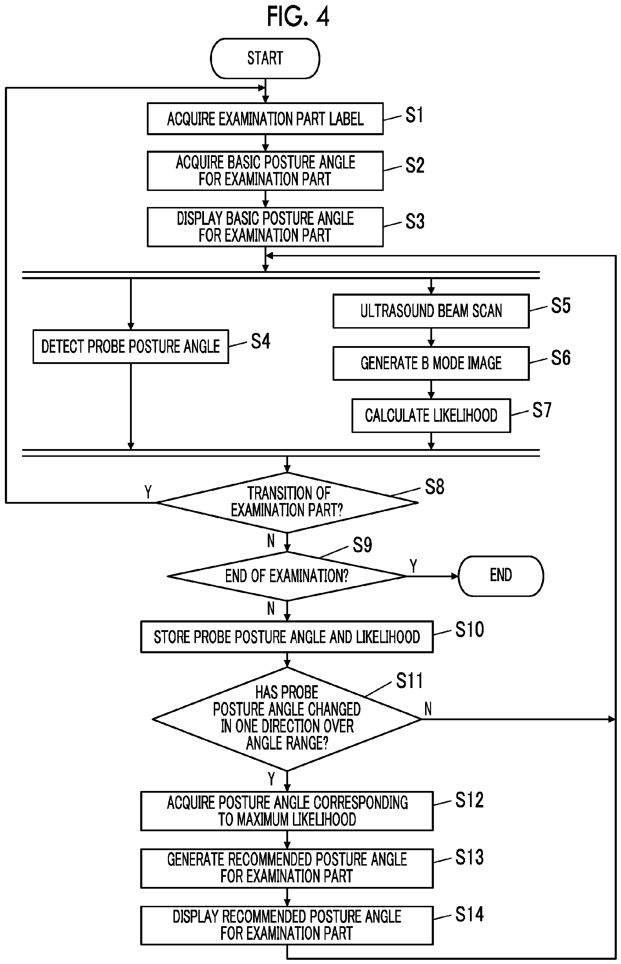

Next, the operation of the ultrasound diagnostic apparatus 1 in the first embodiment will be described with reference to the flowchart shown in Fig. 4. Here, it is assumed that the examination part label and the basic posture angle are stored in advance in the memory 16.

First, in step S1, the apparatus control unit 13 acquires an examination part label for designating a part of a subject, which is to be imaged in the following steps, from the memory 16. Thus, in the following steps, each unit of the ultrasound diagnostic apparatus 1 performs processing corresponding to the examination part designated by the examination part label.

Here, in continuous diagnosis such as so-called eFAST examination, an examination protocol that is the order of examination parts to be subjected to ultrasound diagnosis in examination facilities and the like is often determined in advance in many cases. Therefore, in the operation of the ultrasound diagnostic apparatus 1 to be described below, description will be given on the assumption that the examination part label sequentially transitions according to a predetermined examination protocol.

In step S2, the apparatus control unit 13 further acquires a basic posture angle corresponding to the examination part, which is designated by the examination part label acquired in step S1, from the memory 16.

In subsequent step S3, the apparatus control unit 13 transmits, to the display control unit 7, instruction information for displaying the basic posture angle acquired in step S2 on the display unit 8, and the display control unit 7 displays the basic posture angle on the display unit 8. In the following steps, the operator brings the ultrasound probe 2 into contact with the body surface of the subject and tilts the ultrasound probe 2 with reference to the basic posture angle displayed on the display unit 8, thereby being able to capture an ultrasound image.

In step S4, while the operator brings the ultrasound probe 2 into contact with the body surface of the subject and tilts the ultrasound probe 2, the posture angle of the ultrasound probe 2, that is, the pitch angle, the roll angle, and the yaw angle of the ultrasound probe 2 are detected by the probe posture angle detection unit 9.

In step S5, scanning and transmission and reception of ultrasound beams using a plurality of ultrasound transducers of the transducer array 2A of the ultrasound probe 2, that is, capturing of an ultrasound image, is performed by the reception circuit 4 and the transmission circuit 5 of the image acquisition unit 3. In this case, a reception signal is output to the reception circuit 4 from each ultrasound transducer that has received the ultrasound echo from the subject, and amplification and A/D conversion of the reception signal are performed by the amplification unit 17 and the A/D conversion unit 18 of the reception circuit 4 to generate a reception signal.

In subsequent step S6, the reception signal is input to the image generation unit 6, and a B mode image, that is, an ultrasound image is generated by the B mode processing unit 19 of the image generation unit 6.

Then, in a case where the ultrasound image generated in step S6 is input to the image analysis unit 10, image analysis on the ultrasound image is performed by the image analysis unit 10 and as a result, the likelihood of the imaging part in the ultrasound image with respect to the examination part is calculated in step S7. In this case, for example, the image analysis unit 10 can perform pattern recognition, such as a template matching, calculate the similarity between the part included in the ultrasound image and the template of a plurality of parts as a score, and sets the calculated score as the likelihood of the imaging part in the ultrasound image.

The above-described steps S4 and S5 to S7 are performed synchronously and in parallel. That is, by the processing of steps S4 to S7, the posture angle of the ultrasound probe 2 at the time of generating the ultrasound image by the image generation unit 6 and the likelihood of the imaging part in the generated ultrasound image can be obtained in parallel.

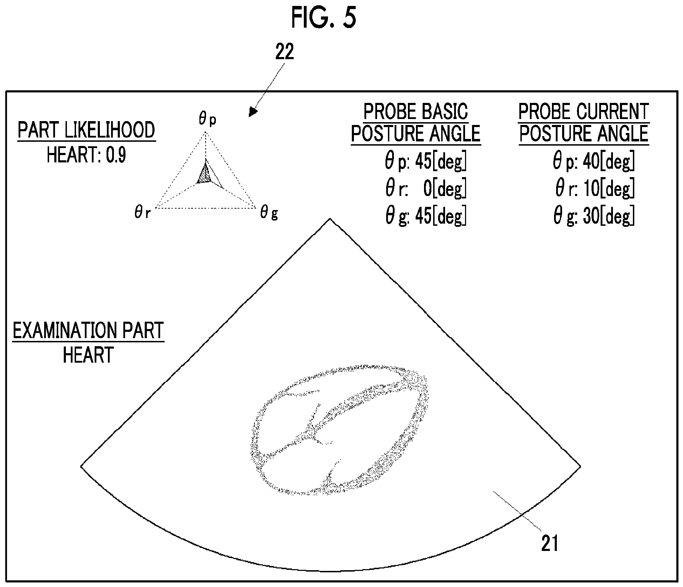

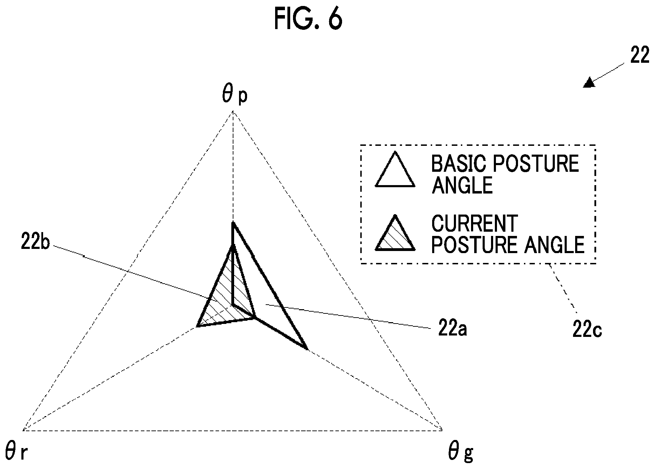

Upon completion of the processing of steps S4 to S7, the posture angle of the ultrasound probe 2 detected in step S4 and the likelihood calculated in step S7 are displayed together with the ultrasound image generated in step S6 on the display unit 8, for example, as shown in Fig. 5. Fig. 5 shows a display example on the display unit 8 in a case where the heart is imaged. In Fig. 5, the likelihood of an imaging part with respect to the heart is displayed as a part likelihood together with an ultrasound image 21, it is displayed that the examination part is the heart, and the basic posture angle and the current posture angle of the ultrasound probe 2 are displayed using numerical values as a pitch angle θp, a roll angle θr, and a yaw angle θg and a radar chart 22. In the radar chart 22, for example, as shown in Fig. 6, a graph 22a for the basic posture angle and a graph 22b for the posture angle of the current ultrasound probe 2 are displayed with the pitch angle θp, the roll angle θr, and the yaw angle θg as three axes. An annotation image 22c for explaining the two graphs 22a and 22b displayed within the radar chart 22 can also be displayed on the radar chart 22.

As described above, the operator can clearly image the examination part of the subject according to the basic posture angle by tilting the ultrasound probe 2 so that the radar chart and the numerical value of the current posture angle of the ultrasound probe 2 match the radar chart and the numerical value of the basic posture angle.

In step S8 subsequent to steps S4 to S7, the apparatus control unit 13 determines whether or not the examination part of the subject being currently examined has transitioned to another part. For example, in a case where the examination part transitions from the heart to the lung, the apparatus control unit 13 determines that the examination part has been changed. In general, in a case where the examination part is changed, the ultrasound probe 2 is separated from the body surface of the subject to become in an air radiation state. By detecting the air radiation state, that is, a state in which the reflection signal from the transducer array 2A cannot be obtained in the reception circuit 4, it is possible to determine the presence or absence of transition of the examination part. In a case where it is determined that the examination part has transitioned in step S8, the process returns to step S1 to acquire a new examination part label according to the predetermined examination protocol, and the processing of steps S2 to S7 is newly performed thereafter.

In a case where it is determined that the examination part has not been changed, that is, the same examination part is examined in step S8, the process proceeds to step S9.

In step S9, the apparatus control unit 13 determines whether or not to end the examination being currently performed. For example, in a case where the operator inputs instruction information for ending the examination to the apparatus control unit 13 through the operation unit 14, the apparatus control unit 13 determines that the examination being currently performed is to be ended. In a case where the apparatus control unit 13 determines that the examination is to be ended as described above, the current examination in the ultrasound diagnostic apparatus 1 is ended.

For example, in a case where the operator does not input instruction information for ending the examination through the operation unit 14, the apparatus control unit 13 determines that the examination being currently performed is not to be ended. In a case where the apparatus control unit 13 determines that the examination is not to be ended as described above, the process proceeds to step S10.

In step S10, the apparatus control unit 13 stores the posture angle of the ultrasound probe 2 detected in step S4 and the likelihood of the imaging part calculated in step S7 in the memory 16.

In subsequent step S11, the apparatus control unit 13 determines whether or not the posture angle of the ultrasound probe 2 detected in step S4 has changed once over a predetermined angle range including the basic posture angle displayed in step S3. Here, "the posture angle of the ultrasound probe 2 changes once over the predetermined angle range" means that a series of changes, such as a change in the posture angle of the ultrasound probe 2 from a value exceeding the upper limit of the predetermined angle range to a value in the angle range and a change from the value in the angle range to a value less than the lower limit of the angle range, are made once. In addition, similarly to the above, "the posture angle of the ultrasound probe 2 changes once over the predetermined angle range" refers to one-time change from a value less than the lower limit of the angle range to a value exceeding the upper limit.

In step S11, in a case where the apparatus control unit 13 determines that the posture angle of the ultrasound probe 2 has not changed in one direction as described above over the predetermined angle range including the basic posture angle displayed in step S3, the process returns to step S4 and step S5. In this case, generation of an ultrasound image, detection of the posture angle of the ultrasound probe 2, and calculation of the likelihood of an imaging part are newly performed, and the newly detected posture angle of the ultrasound probe 2 and the newly calculated likelihood of the imaging part are newly stored in the memory 16. Thus, as a result of the tilting of the ultrasound probe 2 by the operator while repeating the steps S4 to S10, in a case where the apparatus control unit 13 determines that the posture angle of the ultrasound probe 2 has changed in one direction as described above over the predetermined angle range, the process proceeds to step S12.

In step S12, the apparatus control unit 13 acquires a maximum likelihood among the plurality of likelihoods stored in the memory 16 in a case where the processing of steps S4 to S11 is repeated, and acquires the posture angle of the ultrasound probe 2 corresponding to the maximum likelihood, that is, the posture angle of the ultrasound probe 2 detected in synchronization with the maximum likelihood.

In subsequent step S13, the recommended posture angle generation unit 11 sets the posture angle of the ultrasound probe 2 acquired in step S12 as a recommended posture angle for the current examination part.

In subsequent step S14, the recommended posture angle notification unit 12 transmits, to the display control unit 7, instruction information for displaying the recommended posture angle acquired in step S13 on the display unit 8 instead of the basic posture angle. As a result, on the display unit 8, the recommended posture angle is displayed at the location where the basic posture angle is displayed.

Upon completion of step S14, the process returns to step S4 and step S5. In the subsequent processing, as long as the examination part does not transition in step S8 or as long as the end of the examination is not determined in step S9, the processing of steps S4 to S14 is repeated. At this time, in a case where a likelihood greater than the likelihood of the imaging part corresponding to the recommended posture angle acquired in step S13 is calculated, the maximum likelihood is updated in step S12, the posture angle of the ultrasound probe 2 corresponding to the updated likelihood is set as a new recommended posture angle in step S13, and the new recommended posture angle is displayed on the display unit 8 in step S14.

As described above, according to the first embodiment of the present invention, it is possible to obtain a recommended posture angle that is an optimal posture angle of the ultrasound probe 2 at which the examination part of the subject can be clearly imaged in accordance with the structure, the position, and the like of the part of the subject, and the operator can be notified of the recommended posture angle by displaying the recommended posture angle on the display unit 8. Therefore, the operator can clearly image the part even in a case where the subject is changed by capturing an ultrasound image while referring to the recommended angle displayed on the display unit 8.

Although the case is exemplified in which an angle sensor is used as the probe posture angle detection unit 9, the probe posture angle detection unit 9 is not particularly limited as long as the posture angle of the ultrasound probe 2 can be detected. For example, an acceleration sensor, a gyro sensor, a magnetic sensor, a global positioning system (GPS) sensor, and the like can be used as the probe posture angle detection unit 9. In this case, it is possible to provide an apparatus that converts an electric signal obtained from each sensor into the posture angle of the ultrasound probe 2 using a known calculation method. These sensors may be mounted on the ultrasound probe 2 or may be built into the ultrasound probe 2.

In step S1 of Fig. 4, the examination part label is acquired according to the examination protocol, but the method of acquiring the examination part label is not particularly limited. That is, the examination part label can also be input to the operator through the operation unit 14, or can also be determined by performing part determination by the ultrasound diagnostic apparatus 1.

In a case where the examination part label is determined by part determination, it is possible to perform part determination using image analysis, for example. In this case, for example, prior to acquiring the examination part label, an ultrasound image can be generated by the same processing as in steps S5 and S6, likelihoods of respective imaging parts with respect to a plurality of examination parts can be calculated by analyzing the generated ultrasound image, and an examination part corresponding to the maximum likelihood among the likelihoods can be set as a part label. In this case, for example, in the display example shown in Fig. 5, the likelihood of each imaging part with respect to a plurality of examination parts may be displayed in the part likelihood field displayed on the display unit 8, or the examination part field may be displayed as a determination result field and the examination part determined as the examination label in the field may be displayed. The part determination is not limited to the method described above. For example, the apparatus control unit 13 can perform part determination by a known method using a result of motion analysis or the like on imaging parts in a plurality of captured ultrasound images.

In the display example shown in Fig. 5, an image showing the relationship between the ultrasound probe 2 and the x axis, the y axis, and the z axis and the relationship between the ultrasound probe 2 and the pitch angle θp, the roll angle θr, and the yaw angle θg can also be added. In this case, the operator can easily check how the pitch angle θp, the roll angle θr, and the yaw angle θg displayed on the display unit 8 are defined. Therefore, even an operator unfamiliar with the ultrasound diagnostic apparatus 1 can clearly image the examination part of the subject relatively easily by tilting the ultrasound probe 2 while referring to the display on the display unit 8.

In steps S3 and S14, in order to notify the operator of the basic posture angle and the recommended posture angle, the apparatus control unit 13 and the recommended posture angle notification unit 12 transmit, to the display control unit 7, instruction information for displaying the basic posture angle and the recommended posture angle on the display unit 8. However, the notification method of the basic posture angle and the recommended posture angle is not particularly limited to the method. For example, the recommended posture angle notification unit 12 and the apparatus control unit 13 can also notify the operator of the basic posture angle and the recommended posture angle by sound. In addition, apart from the recommended posture angle notification unit 12 and the apparatus control unit 13, it is also possible to provide an apparatus for notifying the operator of the basic posture angle and the recommended posture angle using a sound.

In the display examples shown in Figs. 5 and 6, the basic posture angle and the current posture angle of the ultrasound probe 2 are displayed using the radar chart 22. However, it is needless to say that the basic posture angle and the current posture angle of the ultrasound probe 2 can also be displayed using other display methods. For example, it is also possible to display the basic posture angle and the posture angle of the ultrasound probe 2 using a graph image other than the radar chart 22, such as a bar graph.

Alternatively, a model of an ultrasound probe can be displayed so as to be superimposed on a schema that is a two-dimensional display of a model of the human body, and a guide showing a rotation direction aiming at the basic posture angle with respect to the current posture angle of the ultrasound probe 2 can be displayed. The guide display may be displayed using an image, such as an arrow, or may be accompanied by sound.

The posture angle of the ultrasound probe 2 includes a pitch angle, a roll angle, and a yaw angle, but guidance can be performed using screen display, sound, and the like for each component of the three components, or can be performed at the same time for a plurality of components.

Needless to say, the same display method and the guide method as for the basic posture angle can be applied to the recommended posture angle.

In step S6, the B mode image is generated using the element data obtained by ultrasound beam scanning in step S5. However, measurement information, such as the Doppler information and modulus-of-elasticity information of the imaging part, can be displayed so as to be superimposed on the B mode image. For example, a Doppler image generation unit is provided in the ultrasound diagnostic apparatus 1, Doppler measurements, such as color Doppler measurement and power Doppler measurement, are performed at the time of ultrasound beam scanning in step S5, and a colored image obtained by the Doppler measurement is displayed so as to be superimposed on the B mode image, so that it is possible to generate a Doppler image, such as a color Doppler image or a power Doppler image. In addition, for example, at the time of ultrasound beam scanning in step S5, the modulus of elasticity of the imaging part can be measured. In this case, for example, a modulus-of-elasticity image generation unit is provided in the ultrasound diagnostic apparatus 1, and a modulus-of-elasticity map obtained by elasticity measurement is displayed so as to be superimposed on the B mode image, so that it is possible to generate a modulus-of-elasticity image.

In addition, the likelihood of the imaging part is calculated by performing pattern recognition on the B mode image generated in step S6. However, the present invention is not limited to the pattern recognition as long as the likelihood of the imaging part can be calculated. For example, in a case where measurement, such as Doppler measurement and modulus-of-elasticity measurement, is performed on the imaging part at the time of ultrasound beam scanning in step S5, the likelihood of the imaging part can also be calculated by analyzing the measurement results. For example, in a case where Doppler measurements, such as color Doppler measurement and power Doppler measurement, are performed on the imaging part, the likelihood of the imaging part can be calculated by analyzing the Doppler information, such as the distribution of the blood flow and the speed of the blood flow in the imaging part, and referring to the look-up table stored in advance in the storage unit 15 or the memory 16.

Steps S4 and S5 to S7 are performed synchronously and in parallel. However, steps S4 and S5 to S7 may not be performed synchronously and in parallel as long as the posture angle of the ultrasound probe 2 detected in step S4 and the likelihood of the imaging part calculated in step S7 can be associated with each other. That is, as long as the posture angle detected in step S4 and the likelihood calculated in step S7 are stored so as to be associated with each other in step S10, steps S5 to S7 may be performed immediately after step S4 is completed, or step S4 may be performed immediately after step S7 is completed.

In addition, the posture angle of the ultrasound probe 2 detected in step S4 and the likelihood of the imaging part calculated in step S7 are stored in the memory 16 in step S10. However, the timing at which the detected posture angle of the ultrasound probe 2 and the calculated likelihood of the imaging part are stored may be any timing as long as the timing is in a range from the completion time of step S4 and step S7 to immediately before step S11. For example, the posture angle of the ultrasound probe 2 detected immediately after completion of step S4 can be stored in the memory 16, and the likelihood of the imaging part can be stored in the memory 16 immediately after completion of step S7.

The angle range used in step S11 is not particularly limited as long as the basic posture angle is included therein. In a case where the posture angle of the ultrasound probe 2 is changed with the basic posture angle as a reference, it is preferable to use a predetermined angle range having a certain range on the upper limit side and the lower limit side with the basic posture angle as the center. In this case, in a case where step S11 is performed again without transition of the examination part after the recommended posture angle is obtained in step S13, it is preferable to use a predetermined angle range having a recommended posture angle at the center instead of the basic posture angle. In this manner, by changing the posture angle of the ultrasound probe 2 around a more appropriate recommended posture angle, it is possible to obtain a recommended posture angle at which the examination part of the subject can be more clearly imaged each time the processing of steps S4 to S14 is repeated.

Incidentally, in step S11, the apparatus control unit 13 can perform determination using one predetermined angle, that is, the basic posture angle acquired in step S2, instead of performing determination as to whether or not the posture angle of the ultrasound probe 2 detected in step S4 has changed once over an angle range that includes the basic posture angle and has predetermined different upper and lower limits.

Here, instead of step S11, in a case where the apparatus control unit 13 performs determination using the basic posture angle, the apparatus control unit 13 determines whether or not the posture angle of the ultrasound probe 2 detected in step S4 has changed in one direction across the basic posture angle. That is, the apparatus control unit 13 determines whether or not the posture angle of the ultrasound probe 2 has changed from an angle less than the basic posture angle to an angle exceeding the basic posture angle or determines whether or not the posture angle of the ultrasound probe 2 has changed from an angle exceeding the basic posture angle to an angle less than the basic posture angle.

In this step, in a case where the apparatus control unit 13 determines that the posture angle of the ultrasound probe 2 has not changed in one direction across the basic posture angle, the process returns to step S4 and step S5. On the other hand, in a case where the apparatus control unit 13 determines that the posture angle of the ultrasound probe 2 has changed in one direction across the basic posture angle, the process proceeds to step S12.

In a case where generation and display of a recommended posture angle are performed in subsequent steps S13 and S14 and a step is performed to determine again whether or not the posture angle of the ultrasound probe 2 has changed in one direction across one predetermined angle, the recommended posture angle generated in step S13 is used as the one predetermined angle.

As described above, also in the case of performing a step of determining whether or not the posture angle of the ultrasound probe 2 has changed in one direction across one predetermined angle instead of step S11 of determining whether or not the posture angle of the ultrasound probe 2 has changed once over the predetermined angle range including the basic posture angle, it is possible to obtain a recommended posture angle that is an optimum posture angle of the ultrasound probe 2 at which the examination part of the subject can be clearly imaged in accordance with the structure, the position, and the like of the part of the subject.

Since the ultrasound diagnostic apparatus 1 described above is small, the ultrasound diagnostic apparatus 1 may be a portable ultrasound diagnostic apparatus that can be easily carried and used, or may be a stationary ultrasound diagnostic apparatus that is installed and used in the examination room or the like.

The ultrasound probe 2 is not particularly limited as long as transmission of an ultrasound beam toward the subject and reception of an ultrasound beam reflected from the subject are possible, and may be in the form of a sector type, a convex type, a linear type, a radial type, or the like.

### Second embodiment

In the first embodiment, as shown in Fig. 4, the posture angle of the ultrasound probe 2 at which the likelihood of the imaging part is maximized in a case where the posture angle of the ultrasound probe 2 detected by the probe posture angle detection unit 9 has changed in one direction over the predetermined angle range is set as a recommended posture angle. However, in order to improve the clarity of the imaging part in the ultrasound image, that is, in order to acquire a more appropriate posture angle for the subject, it is also possible to change the posture angle of the ultrasound probe 2 detected by the probe posture angle detection unit 9 in a reciprocating manner over the predetermined angle range.

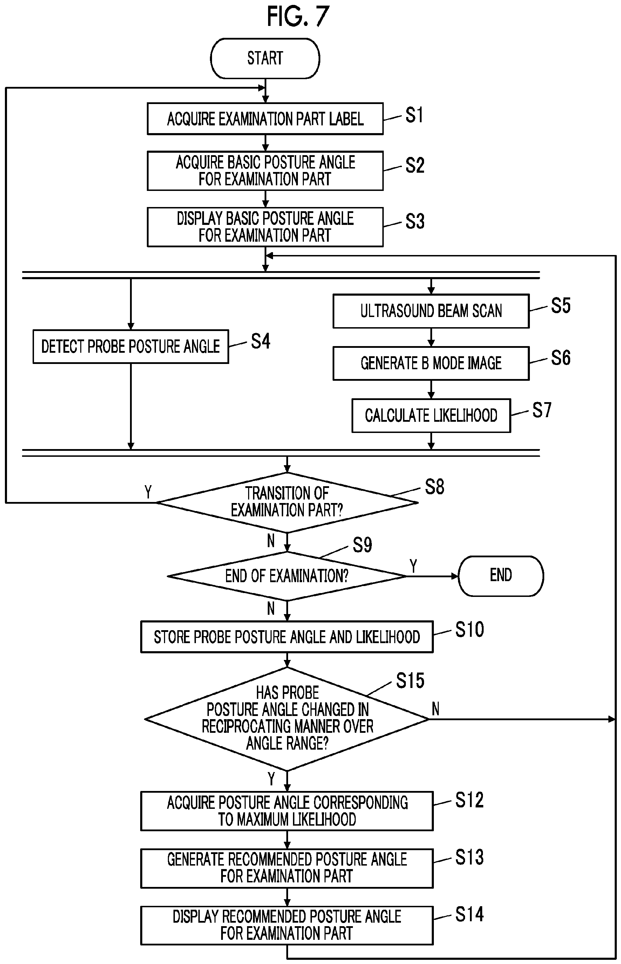

The flowchart of Fig. 7 shows the operation of an ultrasound diagnostic apparatus according to a second embodiment of the present invention. The ultrasound diagnostic apparatus of the second embodiment is the same as the ultrasound diagnostic apparatus 1 of the first embodiment shown in Figs. 1 to 3. Therefore, in the following description, the same reference numerals as for the elements of the ultrasound diagnostic apparatus 1 are used, and the detailed description of each element will be omitted.

Steps S1 to S10 in the second embodiment shown in Fig. 7 are the same as steps S1 to S10 in the first embodiment shown in Fig. 4. After acquisition of the examination part label, acquisition and display of the basic posture angle, detection of the posture angle of the ultrasound probe 2, generation of the ultrasound image, and calculation of the likelihood of the imaging part, determination as to whether or not the examination part has transitioned and determination as to whether or not to end the examination are performed. In a case where the posture angle of the ultrasound probe 2 detected in step S4 and the likelihood of the imaging part calculated in step S7 are stored in the memory 16 in step S10, the process proceeds to step S15.

In step S15, the apparatus control unit 13 determines whether or not the posture angle of the ultrasound probe 2 detected in step S4 has changed in a reciprocating manner over a predetermined angle range including the basic posture angle displayed in step S3. Here, "the posture angle of the ultrasound probe 2 changes in a reciprocating manner over a predetermined angle range" means that the posture angle of the ultrasound probe 2 changes from a value less than the lower limit of the predetermined angle range to a value exceeding the upper limit immediately after changing from a value exceeding the upper limit of the predetermined angle range to a value less than the lower limit or, on the contrary, that the posture angle of the ultrasound probe 2 changes from a value less than the lower limit of the predetermined angle range so as to return to the value less than the lower limit through the value exceeding the upper limit.

In step S15, in a case where the apparatus control unit 13 determines that the posture angle of the ultrasound probe 2 has not changed in a reciprocating manner over the predetermined angle range including the basic posture angle displayed in step S3, the process returns to step S4 and step S5. In this case, as in the first embodiment, generation of an ultrasound image, detection of the posture angle of the ultrasound probe 2, and calculation of the likelihood of an imaging part are newly performed, and the newly detected posture angle of the ultrasound probe 2 and the newly calculated likelihood of the imaging part are newly stored in the memory 16. Thus, as a result of the tilting of the ultrasound probe 2 by the operator while repeating the steps S4 to S10, in a case where the apparatus control unit 13 determines that the posture angle of the ultrasound probe 2 has changed in a reciprocating manner over the predetermined angle range, the process proceeds to step S12.

Subsequent steps S12 to S14 are the same as steps S12 to S14 in the first embodiment shown in Fig. 4. That is, the posture angle of the ultrasound probe 2 corresponding to the maximum likelihood among the plurality of likelihoods stored in the memory 16 is set as a recommended posture angle and the recommended posture angle is displayed on the display unit 8, and the process returns to step S4 and step S5.

As described above, by changing the posture angle of the ultrasound probe 2 in a reciprocating manner over the predetermined angle range rather than changing the posture angle of the ultrasound probe 2 in one direction over the predetermined angle range, it is possible to increase the sampling number of the likelihood of the imaging part and the posture angle of the ultrasound probe 2 in the predetermined angle range. Therefore, according to the second embodiment of the present invention, since the probability of acquiring a more appropriate recommended posture angle for the subject can be increased, the operator can image the examination part of the subject more clearly.

Also in the second embodiment described above, as in the first embodiment, determination using one predetermined angle can be performed instead of performing determination using an angle range having predetermined different upper and lower limits as in step S15. In a case where the predetermined angle is the basic posture angle acquired in step S2, in a step (not shown) replacing step S15, the apparatus control unit 13 determines whether or not the posture angle of the ultrasound probe 2 detected in step S4 has changed in a reciprocating manner across the basic posture angle. That is, the apparatus control unit 13 determines whether or not the posture angle of the ultrasound probe 2 has changed from an angle less than the basic posture angle to an angle exceeding the basic posture angle and has changed from an angle exceeding the basic posture angle to an angle less than the basic posture angle.

In this step, in a case where the apparatus control unit 13 determines that the posture angle of the ultrasound probe 2 has not changed in a reciprocating manner across the basic posture angle, the process returns to step S4 and step S5. On the other hand, in a case where the apparatus control unit 13 determines that the posture angle of the ultrasound probe 2 has changed in a reciprocating manner across the basic posture angle, the process proceeds to step S12.

### Third embodiment

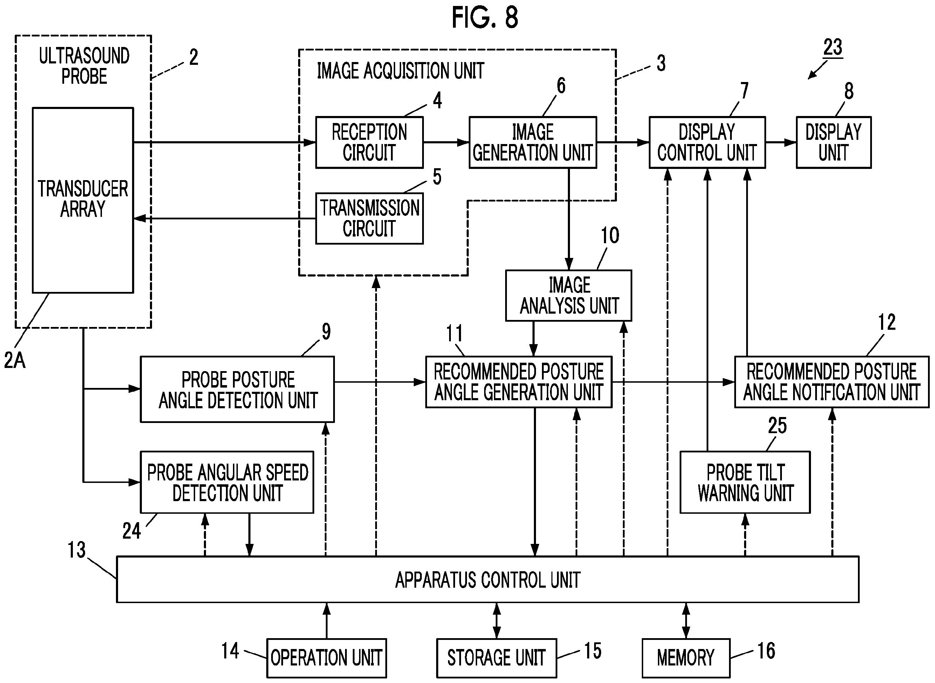

Fig. 8 shows the configuration of an ultrasound diagnostic apparatus according to a third embodiment of the present invention. An ultrasound diagnostic apparatus 23 shown in Fig. 8 is the same as the ultrasound diagnostic apparatus 1 shown in Figs. 1 to 3 except that a probe angular speed detection unit 24 and a probe tilt warning unit 25. Therefore, in the ultrasound diagnostic apparatus 23, the same reference numerals are used for the same elements as in the ultrasound diagnostic apparatus 1, and the detailed description of each element will be omitted.

As shown in Fig. 8, the ultrasound probe 2 comprises the probe angular speed detection unit 24, and the apparatus control unit 13 is connected to the probe angular speed detection unit 24. The probe tilt warning unit 25 is connected to the apparatus control unit 13, and the probe tilt warning unit 25 is connected to the display control unit 7.

The probe angular speed detection unit 24 detects the angular speed of the ultrasound probe 2 that is brought into contact with the body surface of the subject and tilted by the operator. In this case, the probe angular speed detection unit 24 detects the angular speeds of three components centered on three axes perpendicular to each other in a three-dimensional space. For example, it is possible to detect three angular speeds centered on the x axis, the y axis, and the z axis described above with respect to the ultrasound probe 2. In the following description, it is assumed that the probe angular speed detection unit 24 detects the three angular speeds of three components centered on the x axis, the y axis, and the z axis.

The probe angular speed detection unit 24 is not particularly limited as long as the angular speed of the ultrasound probe 2 can be detected. Although not shown, the following description will be given on the assumption that the probe angular speed detection unit 24 is attached to the ultrasound probe 2, includes a gyro sensor that detects the angular speeds of three components centered on the x axis, the y axis, and the z axis as electric signals, and detects angular speed information of the ultrasound probe 2 by converting the electric signal obtained from the gyro sensor into angular speed information.

The probe tilt warning unit 25 warns the operator that the ultrasound probe 2 is to be continuously tilted in a case where the angular speed of the ultrasound probe 2 detected by the probe angular speed detection unit 24 becomes equal to or less than a determined value. Although The probe tilt warning unit 25 can warn the operator that the ultrasound probe 2 is to be continuously tilted using various methods, the following description will be given on the assumption that the probe tilt warning unit 25 transmits, to the display control unit 7, instruction information for displaying a text to continuously tilt the ultrasound probe 2 on the display unit 8.

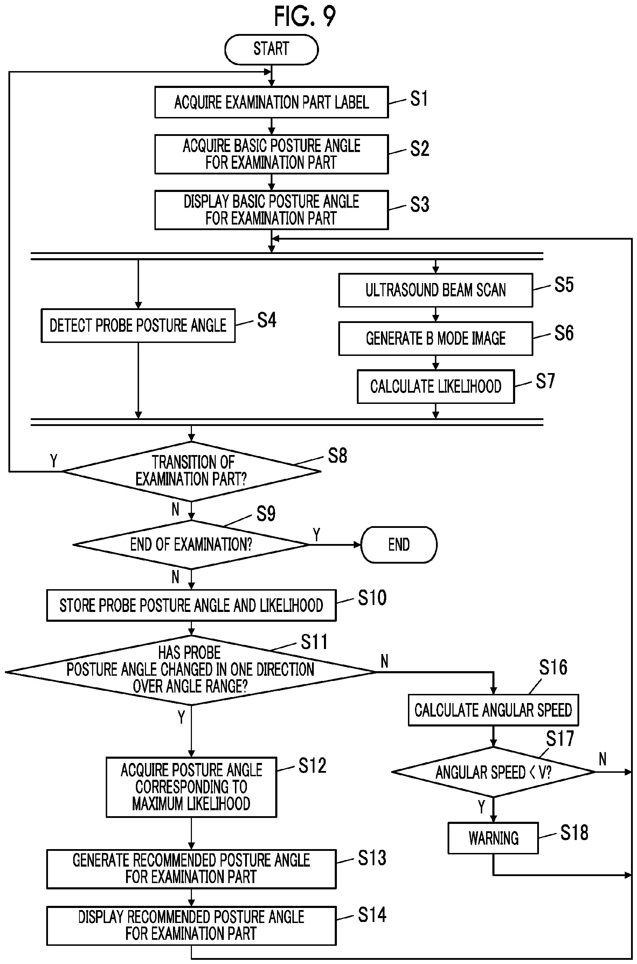

Next, the operation of the ultrasound diagnostic apparatus 23 according to the third embodiment will be described with reference to the flowchart shown in Fig. 9.

Steps S1 to S11 are the same as steps S1 to S11 in the flowchart according to the first embodiment shown in Fig. 4. Acquisition of the examination part label, acquisition of the basic posture angle, display of the basic posture angle on the display unit 8, detection of the posture angle of the ultrasound probe 2, generation of the ultrasound image, and calculation of the likelihood of the examination part are performed, and determination as to whether or not the examination part has transitioned and determination as to whether or not to end the examination are performed. In addition, the posture angle detected in step S4 and the likelihood of the imaging part calculated in step S7 are stored in the memory 16, and it is determined whether or not the detected posture angle of the ultrasound probe 2 has changed in one direction over the predetermined angle range.

In this case, in a case where the apparatus control unit 13 determines that the posture angle of the ultrasound probe 2 detected in step S4 has not changed in one direction over the predetermined angle range in step S11, the process proceeds to step S16.

In step S16, while the operator brings the ultrasound probe 2 into contact with the body surface of the subject and tilts the ultrasound probe 2, the angular speed of the ultrasound probe 2 is detected by the probe angular speed detection unit 24.

In subsequent step S17, the apparatus control unit 13 determines whether or not the posture angle of the ultrasound probe 2 is within a predetermined angle range and whether or not the absolute value of the angular speed of the ultrasound probe 2 detected in step S24 is equal to or less than a predetermined value V. The value V is set to a positive value close to "0", for example. Then, in a case where the apparatus control unit 13 determines that the angular speed of the ultrasound probe 2 exceeds the predetermined value V, it is determined that the ultrasound probe 2 is in contact with the body surface of the subject and is continuously tilted, and the process returns to step S4 and step S5. On the other hand, in a case where it is determined that the absolute value of the angular speed of the ultrasound probe 2 is equal to or less than the predetermined value V, it is determined that the tilting of the ultrasound probe 2 by the operator is about to stop, and the process proceeds to step S18.

In step S18, the probe tilt warning unit 25 transmits instruction information, which is for displaying a warning that the ultrasound probe 2 is to be continuously tilted without being stopped on the display unit 8, to the display control unit 7 under the control of the apparatus control unit 13. As a result, a warning that the ultrasound probe 2 is to be continuously tilted is displayed on the display unit 8.

Upon completion of step S18, the process returns to step S4 and step S5.

In this manner, as long as it is not determined that the posture angle of the ultrasound probe 2 has changed in one direction over the predetermined angle range in step S11, steps S4 to S11 and steps S16 to S18 are repeated. In a case where it is determined that the posture angle of the ultrasound probe 2 has changed in one direction over the predetermined angle range in step S11, the process proceeds to step S12. Steps S12 to S14 are the same as steps S12 to S14 in the flowchart according to the first embodiment shown in Fig. 4, and the posture angle of the ultrasound probe 2 at which the likelihood of the imaging part is maximized is set as a recommended posture angle and the recommended posture angle is displayed on the display unit 8 instead of the basic posture angle.

As described above, according to the third embodiment of the present invention, in a case where the tilting of the ultrasound probe 2 is about to stop while the ultrasound probe 2 is being tilted by the operator, a warning that the ultrasound probe 2 is to be continuously tilted is given to the operator. As a result, it is possible to promote a change in the posture angle of the ultrasound probe 2, that is, it is possible to promote generation of a recommended posture angle.

A gyro sensor that detects the angular speeds of three components centered on the x axis, the y axis, and the z axis in the ultrasound probe 2 is used as the probe angular speed detection unit 24. However, the probe angular speed detection unit 24 is not particularly limited as long as the angular speed of the ultrasound probe 2 of at least three components can be detected. For example, as the probe angular speed detection unit 24, a plurality of gyro sensors that detect only the angular speed of one component can be used, or a plurality of gyro sensors that detect the angular speeds of two components can be used, or a combination of these gyro sensors can be used. Instead of the gyro sensor, an acceleration sensor, a magnetic sensor, a GPS sensor, and the like can also be used. In this case, it is possible to provide an apparatus that converts an electric signal obtained from each sensor into the angular speed of the ultrasound probe 2 using a known calculation method. These sensors may be mounted on the ultrasound probe 2 or may be built into the ultrasound probe 2.

In addition, a sensor for calculating the posture angle of the ultrasound probe 2 and a sensor for calculating the angular speed of the ultrasound probe 2 can be the same sensor. In this case, a conversion apparatus for converting the electric signal obtained from the sensor into the posture angle of the ultrasound probe 2 and a conversion apparatus for converting the electric signal obtained from the sensor into the angular speed of the ultrasound probe 2 can be provided in the probe posture angle detection unit 9 and the probe angular speed detection unit 24, respectively.

The probe tilt warning unit 25 transmits, to the display control unit 7, instruction information for displaying a text to continuously tilt the ultrasound probe 2 on the display unit 8. However, the warning method of the probe tilt warning unit 25 is not limited thereto as long as a warning that the ultrasound probe 2 is to be continuously tilted can be given to the operator. For example, the probe tilt warning unit 25 can also transmit, to the display control unit 7, instruction information for displaying an image to continuously tilt the ultrasound probe 2 on the display unit 8.

The warning to the operator by the probe tilt warning unit 25 is not limited to that displayed on the display unit 8. For example, an apparatus for generating a sound, such as a speaker, can be provided in the ultrasound diagnostic apparatus 23. In this case, the probe tilt warning unit 25 can generate a sound to continuously tilt the ultrasound probe 2 in the apparatus for generating a sound. In addition, for example, a light source, such as a lamp, can be provided in the ultrasound diagnostic apparatus 23. In this case, the probe tilt warning unit 25 can turn on the lamp or the like as a warning that the ultrasound probe 2 is to be continuously tilted.

The warning of the probe tilt warning unit 25 can include guide information indicating the tilting direction of the ultrasound probe 2. Here, for example, in a case where the posture angle of the ultrasound probe 2 changes from a value exceeding the upper limit of the predetermined angle range to a value within the angle range, the guide information refers to an image such as an arrow and a text for giving an instruction to change the posture angle of the ultrasound probe 2 to a value less than the lower limit of the angle range, a sound, and the like. On the contrary, for example, in a case where the posture angle of the ultrasound probe 2 changes from a value less than the lower limit of the predetermined angle range to a value within the angle range, the guide information refers to an image such as an arrow and a text for giving an instruction to change the posture angle of the ultrasound probe 2 to a value exceeding the upper limit of the angle range, a sound, and the like.

In step S17, the apparatus control unit 13 determines whether or not the absolute value of the angular speed of the ultrasound probe 2 detected in step S16 is equal to or less than the predetermined value V. However, it is preferable that the predetermined value V is a small value so as not to disturb the examination due to excessive warning in step S18.

### Fourth embodiment

In the third embodiment, as shown in Fig. 9, the posture angle of the ultrasound probe 2 at which the likelihood of the imaging part is maximized in a case where the posture angle of the ultrasound probe 2 detected by the probe posture angle detection unit 9 has changed in one direction over the predetermined angle range is set as a recommended posture angle. However, as in the second embodiment described with reference to the flowchart shown in Fig. 7, the posture angle of the ultrasound probe 2 at which the likelihood of the imaging part is maximized in a case where the posture angle of the ultrasound probe 2 detected by the probe posture angle detection unit 9 has changed in a reciprocating manner over the predetermined angle range may be set as a recommended posture angle.

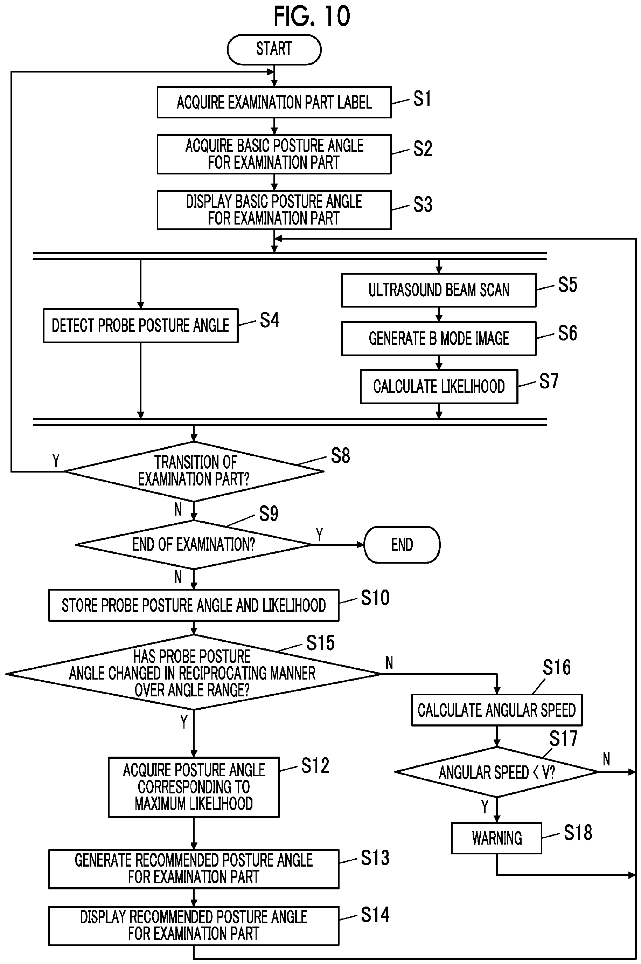

The flowchart of Fig. 10 shows the operation of an ultrasound diagnostic apparatus according to a fourth embodiment. The ultrasound diagnostic apparatus of the fourth embodiment is the same as the ultrasound diagnostic apparatus 23 of the third embodiment shown in Fig. 8. Therefore, in the following description, the same reference numerals as for the elements forming the ultrasound diagnostic apparatus 23 are used, and the detailed description of each element will be omitted. Here, the flowchart shown in Fig. 10 is the same as the flowchart of the third embodiment shown in Fig. 9 except that step S15 is performed instead of step S11. In addition, step S15 in the flowchart of Fig. 10 is the same as step S15 in the flowchart of the second embodiment shown in Fig. 7.

In the fourth embodiment, in a case where the apparatus control unit 13 determines that the posture angle of the ultrasound probe 2 detected in step S4 has not changed in a reciprocating manner over the predetermined angle range in step S15, the process proceeds to step S16. In subsequent steps S16 to S18, the angular speed of the ultrasound probe 2 is detected, and the process returns to step S4 and step S5 in a case where the absolute value of the angular speed exceeds the predetermined value V. On the other hand, in a case where the absolute value of the angular speed of the ultrasound probe 2 is equal to or less than the predetermined value V, a warning that the ultrasound probe 2 is to be continuously tilted is given by the probe tilt warning unit 25, and then the process returns to step S4 and step S5.

Therefore, according to the fourth embodiment of the present invention, rather than changing the posture angle of the ultrasound probe 2 in one direction over the predetermined angle range, it is possible to increase the sampling number of the likelihood of the imaging part and the posture angle of the ultrasound probe 2 in the predetermined angle range. In addition, by warning the operator that the ultrasound probe 2 is to be continuously tilted in a case where the tilting of the ultrasound probe 2 is about to stop while the operator is changing the posture angle of the ultrasound probe 2 in a reciprocating manner over the predetermined angle range, it is possible to promote generation of a recommended posture angle.

While the ultrasound diagnostic apparatus according to the embodiment of the present invention has been described in detail, the present invention is not limited to the above-described embodiments, and various improvements and modifications may be made without departing from the scope and spirit of the present invention. In addition, the plurality of examples shown above can be appropriately used in combination.

### Explanation of References

1, 23: ultrasound diagnostic apparatus
2: ultrasound probe
2A: transducer array
3: image acquisition unit
4: reception circuit
5: transmission circuit
6: image generation unit
7: display control unit
8: display unit
9: probe posture angle detection unit
10: image analysis unit
11: recommended posture angle generation unit
12: recommended posture angle notification unit
13: apparatus control unit
14: operation unit
15: storage unit
16: memory
17: amplification unit
18: A/D conversion unit
19: B mode processing unit
20: image processing unit
21: ultrasound image
22: radar chart
22a, 22b: graph
22c: annotation image
24: probe angular speed detection unit
25: probe tilt warning unit

## Claims

1. An ultrasound diagnostic apparatus (1, 23), comprising:
an ultrasound probe (2);
an image generation unit (6) configured to generate an ultrasound image by transmitting an ultrasound beam from the ultrasound probe (2) toward a subject and receiving an ultrasound beam reflected from the subject;
a display unit (8) configured to display the ultrasound image generated by the image generation unit (6); and
a display control unit (7) configured to control display on the display unit (8);
a probe posture angle detection unit (9) configured to detect a posture angle of the ultrasound probe (2);
an image analysis unit (10) configured to calculate a likelihood of an imaging part in the ultrasound image with respect to an examination part by analyzing the ultrasound image generated by the image generation unit (6); and
a recommended posture angle generation unit (11);
wherein the ultrasound diagnostic apparatus is configured to
display (s2) on the display unit (8) a basic posture angle of the ultrasound probe (2), the basic posture angle being a posture angle of the ultrasound probe (2) set in advance corresponding to the examination part, and being a posture angle of the ultrasound probe (2) at which the examination part of the subject can be imaged so that the imaging part in the ultrasound image becomes clear with respect to the general structure and position of the examination part; and
while the posture angle detected by the probe posture angle detection unit (9) is being changed over a predetermined angle range including the basic posture angle,
detect (s4) the probe posture angle using the probe posture angle detection unit (9) at the time of generating the ultrasound image by the image generation unit;
calculate (s7) a respective likelihood of the imaging part in the generated ultrasound image using the image analysis unit (10);
and
store (s10) the probe posture angle and likelihood; and
after the posture angle has passed over (s11) the predetermined angle range , set (s12, s13), as a recommended posture angle, using the recommended posture angle generation unit (11), the posture angle detected by the probe posture angle detection unit (9) in a case where the likelihood calculated by the image analysis unit (10) is maximized; and the ultrasound diagnostic apparatus further comprises
a recommended posture angle notification unit (12) configured to notify an operator of the recommended posture angle generated by the recommended posture angle generation unit (11) by transmitting instruction information to the display control unit (7) whereby the recommended posture angle is displayed on the display unit (8) instead of the basic posture angle at the location where the basic posture angle is displayed.

2. The ultrasound diagnostic apparatus (1, 23) according to claim 1,
wherein the recommended posture angle generation unit (11) is configured to set, as the recommended posture angle, the posture angle detected by the probe posture angle detection unit (9) in a case where the likelihood calculated by the image analysis unit is maximized while the posture angle detected by the probe posture angle detection unit (9) is changing once over the angle range.

3. The ultrasound diagnostic apparatus (1, 23) according to claim 1,
wherein the recommended posture angle generation unit (11) is configured to set, as the recommended posture angle, the posture angle detected by the probe posture angle detection unit (9) in a case where the likelihood calculated by the image analysis unit is maximized while the posture angle detected by the probe posture angle detection unit (9) is changing in a reciprocating manner over the angle range.

4. The ultrasound diagnostic apparatus (23) according to any one of claims 1 to 3, further comprising:
a probe angular speed detection unit (24) configured to detect an angular speed of the ultrasound probe (2); and
a probe tilt warning unit (25) configured to warn the operator that the ultrasound probe (2) is to be continuously tilted in a case where the posture angle detected by the probe posture angle detection unit (9) is within the angle range and the angular speed detected by the probe angular speed detection unit (24) becomes equal to or less than a predetermined value.

5. The ultrasound diagnostic apparatus (1, 23) according to any one of claims 1 to 4,
wherein the display control unit (7) is configured to display, on the display unit (8), the likelihood calculated by the image analysis unit (10), the posture angle detected by the probe posture angle detection unit (9), the basic posture angle, and the recommended posture angle generated by the recommended posture angle generation unit (11).

6. The ultrasound diagnostic apparatus according to claim 5,
wherein the display control unit is configured to display the posture angle detected by the probe posture angle detection unit, the basic posture angle, and the recommended posture angle generated by the recommended posture angle generation unit, on the display unit, as numerical values or graph images.

7. A control method of an ultrasound diagnostic apparatus (1, 23), comprising:
generating an ultrasound image by transmitting an ultrasound beam from an ultrasound probe (2) toward a subject and receiving an ultrasound beam reflected from the subject;
displaying the ultrasound image generated by the image generation unit (6) on a display unit (8);
displaying (s2) a basic posture angle of the ultrasound probe (2), the basic posture angle being a posture angle of the ultrasound probe (2) set in advance corresponding to the examination part, and being a posture angle of the ultrasound probe (2) at which the examination part of the subject can be imaged so that the imaging part in the ultrasound image becomes clear with respect to the general structure and position of the examination part;
while the posture angle detected by the probe posture angle detection unit (9) is being changed over a predetermined angle range including the basic posture angle,
detecting (s4) a posture angle of the ultrasound probe (2 at the time of generating the ultrasound image by the image generation unit;
calculating (s7) a likelihood of an imaging part in the ultrasound image with respect to an examination part by analyzing the generated ultrasound image; and
storing (s10) the probe posture angle and likelihood; and
after the posture angle has passed over (s11) the predetermined angle range, setting, as a recommended posture angle, the posture angle in a case where the calculated likelihood is maximized; and
notifying an operator of the recommended posture angle by transmitting instruction information to a display control unit (7) whereby the recommended posture angle is displayed on the display unit (8) instead of the basic posture angle at the location where the basic posture angle is displayed.

## Patentansprüche

1. Ultraschalldiagnosegerät (1, 23), umfassend:
eine Ultraschallsonde (2);
eine Bilderzeugungseinheit (6), die so konfiguriert ist, dass sie ein Ultraschallbild erzeugt, indem sie einen Ultraschallstrahl von der Ultraschallsonde (2) in Richtung eines Objektes überträgt und einen Ultraschallstrahl empfängt, der vom Objekt reflektiert wird;
eine Anzeigeeinheit (8), die so konfiguriert ist, dass sie das von der Bilderzeugungseinheit (6) erzeugte Ultraschallbild anzeigt; und
eine Anzeigesteuereinheit (7), die so konfiguriert ist, dass sie die Anzeige auf der Anzeigeeinheit (8) steuert;
eine Sondenhaltungswinkelerfassungseinheit (9), die konfiguriert ist, um einen Haltungswinkel der Ultraschallsonde (2) zu erfassen;
eine Bildanalyseeinheit (10), die konfiguriert ist, um eine Wahrscheinlichkeit eines Bildteils in dem Ultraschallbild in Bezug auf ein Untersuchungsteil zu berechnen, indem das Ultraschallbild analysiert wird, das von der Bilderzeugungseinheit (6) erzeugt wird; und
eine Empfehlungshaltungswinkelerzeugungseinheit (11);
wobei das Ultraschalldiagnosegerät dazu konfiguriert ist,
Anzeigen (s2) auf der Anzeigeeinheit (8) eines Grundhaltungswinkels der Ultraschallsonde (2), wobei der Grundhaltungswinkel ein im Voraus eingestellter Haltungswinkel der Ultraschallsonde (2) entsprechend dem Untersuchungsteil ist, und ein Haltungswinkel der Ultraschallsonde (2) ist, bei dem der Untersuchungsteil des Objektes so abgebildet werden kann, dass der Bildteil im Ultraschallbild in Bezug auf die allgemeine Struktur und Lage des Untersuchungsteils deutlich wird; und
während der von der Sondenhaltungswinkelerfassungseinheit (9) erfasste Haltungswinkel über einen vorbestimmten Winkelbereich einschließlich des Grundhaltungswinkels geändert wird,
Erfassen (s4) des Sondenhaltungswinkels unter Verwendung der Sondenhaltungswinkelerfassungseinheit (9) zum Zeitpunkt der Erzeugung des Ultraschallbildes durch die Bilderzeugungseinheit;
Berechnen (s7) einer jeweiligen Wahrscheinlichkeit des Bildteils in dem erzeugten Ultraschallbild unter Verwendung der Bildanalyseeinheit (10);
Speichern (s10) des Sondenhaltungswinkels und Wahrscheinlichkeit; und
nachdem der Haltungswinkel
nachdem der Haltungswinkel den vorbestimmten Winkelbereich überschritten hat (s11), Festlegen (s12, s13), als ein Empfehlungshaltungswinkel, unter Verwendung der Empfehlungshaltungswinkelerzeugungseinheit (11) des Haltungswinkels, der von der Sondenhaltungswinkelerfassungseinheit (9) erfasst wird, wenn die von der Bildanalyseeinheit (10) berechnete Wahrscheinlichkeit maximiert ist; und das Ultraschalldiagnosegerät ferner umfasst:
eine Empfehlungshaltungswinkelmeldungseinheit (12), die so konfiguriert ist, dass sie einen Bediener über den von der Empfehlungshaltungswinkelerzeugungseinheit (11) erzeugten Empfehlungshaltungswinkel informiert, indem sie Anweisungsinformationen an die Anzeigesteuereinheit (7) sendet, wodurch der Empfehlungshaltungswinkel auf der Anzeigeeinheit (8) anstelle des Grundhaltungswinkels an dem Ort angezeigt wird, an dem der Grundhaltungswinkel angezeigt wird.

2. Ultraschalldiagnosegerät (1, 23) nach Anspruch 1,
wobei die Empfehlungshaltungswinkelerzeugungseinheit (11) so konfiguriert ist, dass sie als Empfehlungshaltungswinkel den Haltungswinkel festlegt, der von der Sondenhaltungswinkelerfassungseinheit (9) in einem Fall erfasst wird, in dem die von der Bildanalyseeinheit berechnete Wahrscheinlichkeit maximiert wird, während sich der von der Sondenhaltungswinkelerfassungseinheit (9) erfasste Haltungswinkel einmal über den Winkelbereich ändert.

3. Ultraschalldiagnosegerät (1, 23) nach Anspruch 1,
wobei die Empfehlungshaltungswinkelerzeugungseinheit (11) so konfiguriert ist, dass sie als Empfehlungshaltungswinkel den Haltungswinkel festlegt, der von der Sondenhaltungswinkelerfassungseinheit (9) in einem Fall erfasst wird, in dem die von der Bildanalyseeinheit berechnete Wahrscheinlichkeit maximiert wird, während sich der von der Sondenhaltungswinkelerfassungseinheit (9) erfasste Haltungswinkel über den Winkelbereich hin- und herbewegt.

4. Ultraschalldiagnosegerät (23) nach einem der Ansprüche 1 bis 3, ferner umfassend:
eine Sondenwinkelgeschwindigkeitserfassungseinheit (24), die so konfiguriert ist, dass sie eine Winkelgeschwindigkeit der Ultraschallsonde (2) erfasst; und
eine Sondenneigungswarneinheit (25), die so konfiguriert ist, dass sie den Bediener warnt, dass die Ultraschallsonde (2) in einem Fall, in dem der von der Sondenhaltungswinkelerfassungseinheit (9) erfasste Haltungswinkel innerhalb des Winkelbereichs liegt und die von der Sondenwinkelgeschwindigkeitserfassungseinheit (24) erfasste Winkelgeschwindigkeit gleich oder kleiner als ein vorbestimmter Wert wird, kontinuierlich zu neigen ist.

5. Ultraschalldiagnosegerät (1, 23) nach einem der Ansprüche 1 bis 4,
wobei die Anzeigesteuereinheit (7) so konfiguriert ist, dass sie auf der Anzeigeeinheit (8) die von der Bildanalyseeinheit (10) berechnete Wahrscheinlichkeit, den von der Sondenhaltungswinkelerfassungseinheit (9) erfassten Haltungswinkel, den Grundhaltungswinkel und den von der Empfehlungshaltungswinkelerzeugungseinheit (11) erzeugten Empfehlungshaltungswinkel anzeigt.

6. Ultraschalldiagnosegerät nach Anspruch 5,
wobei die Anzeigesteuereinheit so konfiguriert ist, dass sie den von der Sondenhaltungswinkelerfassungseinheit erfassten Haltungswinkel, den Grundhaltungswinkel und den von der Empfehlungshaltungswinkelerzeugungseinheit erzeugten Empfehlungshaltungswinkel auf der Anzeigeeinheit als numerische Werte oder grafische Bilder anzeigt.

7. Verfahren zur Steuerung eines Ultraschalldiagnosegeräts (1, 23), umfassend:
Erzeugen eines Ultraschallbildes durch Übertragen eines Ultraschallstrahls von einer Ultraschallsonde (2) zu einem Objekt und Empfangen eines von dem Objekt reflektierten Ultraschallstrahls;
Anzeigen des von der Bilderzeugungseinheit (6) erzeugten Ultraschallbildes auf einer Anzeigeeinheit (8);
Anzeigen (s2) eines Grundhaltungswinkels der Ultraschallsonde (2), wobei der Grundhaltungswinkel ein im Voraus entsprechend dem Untersuchungsteil eingestellter Haltungswinkel der Ultraschallsonde (2) ist und ein Haltungswinkel der Ultraschallsonde (2) ist, bei dem der Untersuchungsteil des Objektes abgebildet werden kann, so dass der Bildteil in dem Ultraschallbild in Bezug auf die allgemeine Struktur und Lage des Untersuchungsteils deutlich wird;
während der von der Sondenhaltungswinkelerfassungseinheit (9) erfasste Haltungswinkel über einen vorbestimmten Winkelbereich einschließlich des Grundhaltungswinkels geändert wird,
Erfassen (s4) eines Haltungswinkels der Ultraschallsonde (2) zum Zeitpunkt der Erzeugung des Ultraschallbildes durch die Bilderzeugungseinheit;
Berechnen (s7) einer Wahrscheinlichkeit eines Bildteils in dem Ultraschallbild in Bezug auf einen Untersuchungsteil durch Analysieren des erzeugten Ultraschallbildes; und
Speichern (s10) des Sondenhaltungswinkels und der Wahrscheinlichkeit; und
nach Überschreiten (s11) des vorbestimmten Winkelbereichs durch den Haltungswinkel, Festlegen des Haltungswinkels als Empfehlungshaltungswinkel in einem Fall, in dem die berechnete Wahrscheinlichkeit maximiert ist; und
Benachrichtigen eines Bedieners über den Empfehlungshaltungswinkel durch Übertragen von Anweisungsinformationen an eine Anzeigesteuereinheit (7), wodurch der Empfehlungshaltungswinkel auf der Anzeigeeinheit (8) anstelle des Grundhaltungswinkels an der Stelle angezeigt wird, an der der Grundhaltungswinkel angezeigt wird.

## Revendications

1. Appareil de diagnostic à ultrasons (1, 23), comprenant :
une sonde ultrasonore (2) ;
une unité de génération d'image (6) configurée pour générer une image échographique en transmettant un faisceau ultrasonore d'une sonde ultrasonore (2) vers un sujet et en recevant un faisceau ultrasonore réfléchi à partir du sujet ;
une unité d'affichage (8) configurée pour afficher l'image échographique générée par l'unité de génération d'image (6), et
une unité de commande d'affichage (7) configurée pour commander l'affichage sur l'unité d'affichage (8) ;
une unité de détection d'angle de posture de sonde (9) configurée pour détecter un angle de posture de la sonde ultrasonore (2) ;
une unité d'analyse d'image (10) configurée pour calculer une probabilité d'une partie d'imagerie dans l'image échographique par rapport à une partie d'examen en analysant l'image échographique générée par l'unité de génération d'image (6), et
une unité de génération d'angle de posture recommandé (11) ;
dans lequel l'appareil de diagnostic à ultrasons est configuré pour
afficher (s2), sur l'unité d'affichage (8), un angle de posture de base de la sonde ultrasonore (2), l'angle de posture de base étant un angle de posture de la sonde ultrasonore (2) réglé à l'avance correspondant à la partie d'examen, et étant un angle de posture de la sonde ultrasonore (2) sur lequel la partie d'examen du sujet peut être imagée de sorte que la partie d'imagerie dans l'image échographique devient claire par rapport à la structure générale et la position de la partie d'examen, et
tandis que l'angle de posture détecté par l'unité de détection d'angle de posture de sonde (9) est en cours de modification sur une plage d'angles prédéterminée incluant l'angle de posture de base,
détecter (s4) l'angle de posture de sonde à l'aide de l'unité de détection d'angle de posture de sonde (9) au temps de la génération de l'image échographique par l'unité de génération d'image ;
calculer (s7) une probabilité respective de la partie d'imagerie dans l'image échographique générée à l'aide de l'unité d'analyse d'image (10), et
stocker (s10) l'angle de posture de sonde et la probabilité, et
après passage de l'angle de posture sur (s11) la plage d'angles prédéterminée, régler (s12, s13), comme angle de posture recommandé, à l'aide de l'unité de génération d'angle de posture recommandé (11), l'angle de posture détecté par l'unité de détection d'angle de posture de sonde (9) dans un cas où la probabilité calculée par l'unité d'analyse d'image (10) est maximisée, et l'appareil de diagnostic à ultrasons comprend en outre :
une unité de notification d'angle de posture recommandé (12) configurée pour notifier un opérateur de l'angle de posture recommandé généré par l'unité de génération d'angle de posture recommandé (11) en transmettant des informations d'instruction à l'unité de commande d'affichage (7), attendu que l'angle de posture recommandé est affiché sur l'unité d'affichage (8), au lieu de l'angle de posture de base à l'endroit où l'angle de posture de base est affiché.

2. Appareil de diagnostic à ultrasons (1, 23) selon la revendication 1,
dans lequel l'unité de génération d'angle de posture recommandé (11) est configurée pour régler, comme angle de posture recommandé, l'angle de posture détecté par l'unité de détection d'angle de posture de sonde (9) dans un cas où la probabilité calculée par l'unité d'analyse d'image est maximisée tandis que l'angle de posture détecté par l'unité de détection d'angle de posture de sonde (9) est en cours de modification une fois sur la plage d'angles.

3. Appareil de diagnostic à ultrasons (1, 23) selon la revendication 1,
dans lequel l'unité de génération d'angle de posture recommandé (11) est configurée pour régler, comme angle de posture recommandé, l'angle de posture détecté par l'unité de détection d'angle de posture de sonde (9) dans un cas où la probabilité calculée par l'unité d'analyse d'image est maximisée tandis que l'angle de posture détecté par l'unité de détection d'angle de posture de sonde (9) est en cours de modification d'une manière réciproque sur la plage d'angles.

4. Appareil de diagnostic à ultrasons (23) selon l'une quelconque des revendications 1 à 3, comprenant en outre :
une unité de détection de vitesse angulaire de sonde (24) configurée pour détecter une vitesse angulaire de la sonde ultrasonore (2), et
une unité d'avertissement de basculement de sonde (25) configurée pour avertir l'opérateur que la sonde ultrasonore (2) est basculée de manière continue dans un cas où l'angle de posture détecté par l'unité de détection d'angle de posture de sonde (9) est compris dans la plage d'angles et la vitesse angulaire détectée par l'unité de détection de vitesse angulaire de sonde (24) devient inférieure ou égale à une valeur prédéterminée.

5. Appareil de diagnostic à ultrasons (1, 23) selon l'une quelconque des revendications 1 à 4,
dans lequel l'unité de commande d'affichage (7) est configurée pour afficher, sur l'unité d'affichage (8), la probabilité calculée par l'unité d'analyse d'image (10), l'angle de posture détecté par l'unité de détection d'angle de posture de sonde (9), l'angle de posture de base, et l'angle de posture recommandé généré par l'unité de génération d'angle de posture recommandé (11).

6. Appareil de diagnostic à ultrasons selon la revendication 5,
dans lequel l'unité de commande d'affichage est configurée pour afficher l'angle de posture détecté par l'unité de détection d'angle de posture de sonde, l'angle de posture de base, et l'angle de posture recommandé généré par l'unité de génération d'angle de posture recommandé, sur l'unité d'affichage, comme valeurs numériques ou images de graphique.

7. Procédé de commande d'un appareil de diagnostic à ultrasons (1, 23), comprenant les étapes consistant à :
générer une image échographique en transmettant un faisceau ultrasonore d'une sonde ultrasonore (2) vers un sujet et en recevant un faisceau ultrasonore réfléchi à partir du sujet ;
afficher l'image échographique générée par l'unité de génération d'image (6) sur une unité d'affichage (8) ;
afficher (s2) un angle de posture de base de la sonde ultrasonore (2), l'angle de posture de base étant un angle de posture de la sonde ultrasonore (2) réglé à l'avance correspondant à la partie d'examen, et étant un angle de posture de la sonde ultrasonore (2) sur lequel la partie d'examen du sujet peut être imagée de sorte que la partie d'imagerie dans l'image échographique devient claire par rapport à la structure générale et la position de la partie d'examen,
tandis que l'angle de posture détecté par l'unité de détection d'angle de posture de sonde (9) est en cours de modification sur une plage d'angles prédéterminée incluant l'angle de posture de base,
détecter (s4) l'angle de posture de la sonde ultrasonore (2) au temps de la génération de l'image échographique par l'unité de génération d'image ;
calculer (s7) une probabilité d'une partie d'imagerie dans l'image échographique par rapport à une partie d'examen en analysant l'image échographique générée, et
stocker (s10) l'angle de posture de sonde et la probabilité, et
après passage de l'angle de posture sur (s11) la plage d'angles prédéterminée, régler, comme angle de posture recommandé, l'angle de posture dans un cas où la probabilité calculée est maximisée, et
notifier un opérateur de l'angle de posture recommandé en transmettant des informations d'instruction à l'unité de commande d'affichage (7), attendu que l'angle de posture recommandé est affiché sur l'unité d'affichage (8), au lieu de l'angle de posture de base à l'endroit où l'angle de posture de base est affiché.
